# EUROPEAN PATENT APPLICATION

(11) **EP 3 098 618 A2**
(43) Date of publication of application: **30.11.2016**
(21) Application number: 16171459.7
(22) Date of filing: 26.05.2016
(51) Int. Cl.: G01R 33/34, G01R 33/341

(54) **RADIO FREQUENCY SURFACE COIL FOR CHEST MAGNETIC RESONANCE IMAGING**

(30) Priority: 27.05.2015 KR 20150073918
(71) Applicant: Samsung Electronics Co., Ltd., Gyeonggi-do 16677 (KR)
(72) Inventor: PARK, Hae-gweon, Gyeonggi-do (KR); PARK, Seul-gi, Gyeonggi-do (KR); LEE, Ju-hyung, Gyeonggi-do (KR); CHANG, Min-soo, Gyeonggi-do (KR)
(74) Representative: Land, Addick Adrianus Gosling

(57) **Abstract**

A radio frequency (RF) surface coil and a magnetic resonance imaging (MRI) apparatus having the RF surface coil are provided. The RF surface coil includes a first cup unit and a second cup unit corresponding to a chest of an object, the first cup unit and the second cup unit being transformable to a shape of the chest. The RF surface coil further includes an RF coil element disposed on the first cup unit and the second cup unit.

## Description

This application claims priority from Korean Patent Application No. 10-2015-0073918, filed on May 27, 2015, in the Korean Intellectual Property Office, the disclosure of which is incorporated herein in its entirety by reference.

Apparatuses consistent with exemplary embodiments relate to radio frequency (RF) surface coils and magnetic resonance imaging (MRI) systems including the same.

For the prevention and control of disease, various diagnosis devices have been used for diagnosing an internal structure of a body under examination. Among such diagnosis devices, an MRI device that uses a magnetic field that is generated from a magnetic force has become widely used.

The MRI device may obtain tomography images of an object by expressing the intensity of a magnetic resonance signal with respect to an RF signal that is generated in a magnetic field of an intensity, by using a brightness contrast. After placing a body to be examined in a strong polarizing magnetic field, when an RF signal that resonates atomic nuclei (for example, hydrogen atomic nuclei) is instantly irradiated to the body to be examined and stopped by using an RF coil, a magnetic resonance signal is generated from the atomic nuclei. The RF coil receives the magnetic resonance signal, and thus, an MRI system may obtain a cross-sectional image of the body to be examined. The intensity of the magnetic resonance signal may be determined according to an amount of predetermined atoms (for example, hydrogen, sodium, carbon isotopes, etc.) that are included in the body or in a stream of blood flow.

Exemplary embodiments may address at least the above problems and/or disadvantages and other disadvantages not described above. Also, the exemplary embodiments are not required to overcome the disadvantages described above, and an exemplary embodiment may not overcome any of the problems described above.

Radio frequency (RF) surface coils that may obtain a magnetic resonance image of a chest of a body to be examined and magnetic resonance imaging (MRI) systems including the RF surface coils, are provided.

According to an aspect of an exemplary embodiment, there is provided a RF surface coil for a magnetic resonance imaging apparatus, the RF surface coil including a first cup unit and a second cup unit corresponding to a chest of an object, the first cup unit and the second cup unit being transformable to a shape of the chest, and an RF coil element disposed on the first cup unit and the second cup unit.

The RF surface coil may further include a center unit disposed between the first cup unit and the second cup unit.

The RF coil element may be further disposed on the center unit.

The RF coil element may be disposed on an inner surface of the first cup unit, the second cup unit, and the center unit.

The RF surface coil may further include a cable connected to the RF coil element.

The RF surface coil may further include a controller connected to the cable, the controller being configured to control the RF coil element to generate an RF magnetic field on the chest of the object, and receive an RF signal that is emitted from the chest.

A length of the center unit may be adjustable.

The center unit may include length control pieces and fixing bands, the length control pieces being fixable by the fixing bands.

The center unit may include a first region and a second region, the first region being separated from the second region.

The RF surface coil may further include a position fixing protrusion disposed in the first region, and a groove disposed in the second region, the position fixing protrusion being moveably inserted in the groove.

The groove may include a first groove in a direction of a length of the center unit, and a second groove in a direction different from the direction of the length of the center unit.

The RF surface coil may further include a first extension unit disposed on an edge of the first cup unit, a second extension unit disposed on an edge of the second cup unit, a first assembling unit disposed on an edge of the first extension unit, and a second assembling unit disposed on an edge of the second extension unit.

At least one among the first extension unit and the second extension unit may include a first region and a second region, the first region having a width different than a width of the second region.

The first assembling unit may be coupled to the second assembling unit.

The first assembling unit may be coupled to the second assembling unit, using one among velcro, a button, and a hook.

At least one among the first cup unit and the second cup unit may include a cover unit transformable according to a shape of the chest of the object.

The at least one among the first cup unit and the second cup unit may include a round unit from which the cover unit extends.

At least one among the cover unit and the round unit may include a flexible material.

The cover unit and the round unit may be one-body.

The cover unit may include cover units, and the cover units and the round unit may be one-body.

The at least one among the first cup unit and the second cup unit may include another cover unit disposed on a center region of an inner surface of the cover units.

The cover unit may include cover units that are individually connected to the round unit. The cover unit may include a non-flexible material.

The at least one among the first cup unit and the second cup unit may include a hinge connecting the cover unit to the round unit.

According to an aspect of another exemplary embodiment, there is provided a magnetic resonance imaging (MRI) apparatus including a cylindrical magnetic structure, and a table on which an object is positioned, the table being configured to be in the cylindrical magnetic structure. The MRI apparatus further includes a radio frequency (RF) surface coil that is worn on a chest of the object.

The table may include a positioning unit on which the chest of the object is positioned, and the positioning unit may include a concave unit disposed in a surface of the positioning unit, the concave unit corresponding to a shape of the chest.

### BRIEF DESCRIPTION OF THE DRAWINGS

The above and/or other aspects will become more apparent by describing exemplary embodiments with reference to the accompanying drawings, in which:
FIG. 1 is a diagram of a magnetic resonance imaging (MRI) system or apparatus, according to an exemplary embodiment;
FIG. 2A is schematic diagram of an object wearing a radio frequency (RF) surface coil, according to an exemplary embodiment;
FIG. 2B is a perspective view of a table on which the object wearing the RF surface coil of FIG. 2A is positioned thereon to capture a magnetic resonance image;
FIG. 2C is a schematic diagram showing an object that is positioned on the table of FIG. 2B and entering into a bore;
FIG. 3A is a bottom view of an RF surface coil, according to an exemplary embodiment;
FIG. 3B is a cross-sectional view of the RF surface coil of FIG. 3A;
FIGS. 4A, 4B, and 4C are schematic diagrams of an RF surface coil in which a length of a center unit is controlled, according to an exemplary embodiment;
FIGS. 5A and 5B are schematic diagrams showing controlling a gap of an RF surface coil, according to an exemplary embodiment;
FIGS. 6A and 6B are schematic diagrams showing controlling a gap of an RF surface coil, according to another exemplary embodiment;
FIGS. 7A and 7B are perspective views of a cup of an RF surface coil, according to an exemplary embodiment;
FIG. 8 is a schematic diagram of a cup of an RF surface coil, according to another exemplary embodiment; and
FIG. 9 is a perspective view of a cup of an RF surface coil, according to another exemplary embodiment.

### DETAILED DESCRIPTION

Exemplary embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments may be practiced without those defined matters. Also, well-known functions or constructions may not be described in detail because they would obscure the description with unnecessary detail.

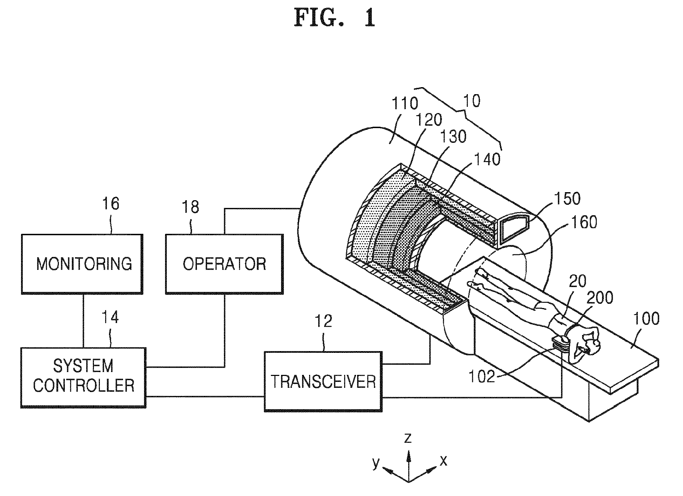

FIG. 1 is a diagram of a magnetic resonance imaging (MRI) system or apparatus, according to an exemplary embodiment.

Referring to FIG. 1, the MRI system according to an exemplary embodiment includes a housing 110, a main magnet 120, a gradient coil 130, and a radio frequency (RF) body coil 140. The housing 110 includes a bore 160 that is an empty space formed in a length direction inside the housing 110, for example, in a z-direction so that a table 100 may enter into the housing 110. A diameter of the bore 160 may be determined according to the sizes of the main magnet 120, the gradient coil 130, and the RF body coil 140. An object 20 to be examined may be positioned on the table 100, and the object 20 may be examined by entering into the bore 160 of the housing 110 in the moving direction of the table 100. The main magnet 120, the gradient coil 130, and the RF body coil 140 respectively may have cylindrical shapes and may constitute a cylindrical magnetic structure 10.

The main magnet 120 may generate a static magnetic field for arranging the direction of a magnetic dipole moment of atomic nuclei of elements, for example, hydrogen, phosphorus, or sodium, that cause a magnetic resonance phenomenon in a direction. For reference, in an exemplary embodiment, the "object" may include a human or an animal, and also, may include parts of a human or an animal. For example, the object 20 may include a heart, liver, brain, breast, uterus, abdominal organ, spinal cord, or blood vessel. The main magnet 120 may be a superconducting magnet or a permanent magnet, for example, a superconducting magnet that may be used for generating a high magnetic field of 0.5 T or greater. The stronger and more uniform a magnetic field generated by the main magnet 120 is, the more relatively precise and correct an obtained magnetic resonance image may be. The main magnet 120 may have a cylindrical shape according to an inner shape of the housing 110.

The gradient coil 130 may be formed inside the main magnet 120 with a cylindrical shape. The gradient coil 130 may include three gradient coils that generate gradient magnetic fields in an x-axis direction, a y-axis direction, and a z-axis direction. To capture a magnetic resonance image, a spatial linear gradient magnetic field may be generated in the gradient coil 130. The gradient coil 130 may provide location information of parts of the object 20 by inducing different resonance frequencies on each part of the object 20.

The RF body coil 140 may have a cylindrical shape, and may be mounted inside the gradient coil 130. The RF body coil 140 may constitute a part of the cylindrical magnetic structure 10 together with the main magnet 120 and the gradient coil 130. An RF coil used in an MRI system may include the RF body coil 140 and an RF surface coil 200. The RF surface coil 200 may have various shapes according to the parts of the object 20. For example, the RF surface coil 200 may have shapes and sizes corresponding to the parts of the object, such as, a torso, head, spine, shoulder, breast knee, ankle, etc. The RF surface coil 200 may be positioned close to the object 20 on the table 100. In an exemplary embodiment, the RF surface coil 200 for obtaining an MRI signal of a chest of the object 20 is provided. A detailed description of the RF surface coil 200 will be described below.

The RF body coil 140 and the RF surface coil 200 may generate an RF magnetic field that uses a Larmor frequency as a center frequency. Also, the RF body coil 140 and the RF surface coil 200 may apply an RF signal to the object 20, and may receive an MRI radio frequency signal, that is, an RF signal emitted from the object 20. The RF body coil 140 and the RF surface coil 200 may generate an electromagnetic signal having a radio frequency corresponding to the type of atomic nucleus, for example, an RF signal, and may apply it to the object 20 to change the energy state of the atomic nucleus from a lower energy state to a higher energy state. When the electromagnetic signal generated from the RF body coil 140 and the RF surface coil 200 dissipates, an electromagnetic wave having a Larmor frequency may be emitted while the energy state of the atomic nucleus is transited to a lower level from a higher level. That is, when the application of an electromagnetic signal with respect to the atomic nucleus is stopped, an electromagnetic wave having a Larmor frequency is emitted to the outside while the energy state of the atomic nucleus to which an RF signal is applied is transited to a lower level from a higher level. The RF body coil 140 and the RF surface coil 200 may receive an electromagnetic signal that is emitted from the atomic nuclei inside the object 20. The RF body coil 140 constitutes a part of the cylindrical magnetic structure 10 and may be fixed on an inner side of the gradient coil 130 of the housing 110. The RF surface coil 200 may be attachably and detachably mounted on the table 100 or the object 20.

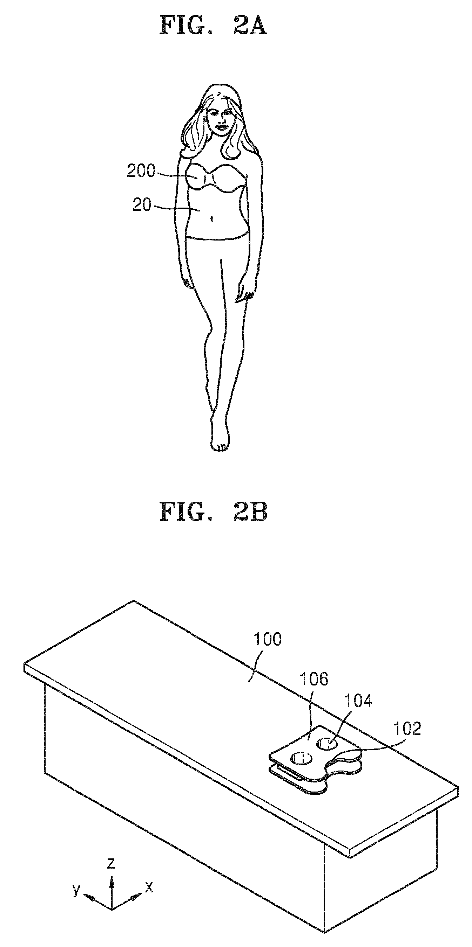

FIG. 2A is schematic diagram of the object 20 wearing the RF surface coil 200, according to an exemplary embodiment.

Referring to FIG. 2A, the RF surface coil 200 may be fixedly mounted on a chest, that is, a breast, of the object 20, and may be readily attached and detached from the chest. To obtain a magnetic resonance image of the object 20, the RF surface coil 200 according to an exemplary embodiment may be mounted to directly contact the chest of the object 20. After mounting the RF surface coil 200 on the chest, the object 20 may be located on the table 100.

FIG. 2B is a perspective view of the table 100 on which the object 20 wearing the RF surface coil 200 of FIG. 2A is positioned thereon to capture a magnetic resonance image.

Referring to FIGS. 2A and 2B, the object 20 wearing the RF surface coil 200 on a chest may be positioned on the table 100, and a positioning unit 102 on which the chest of the object 20 is located is provided on the table 100. The object 20 may lie face down so that the chest to be inspected and diagnosed faces an upper surface of the table 100. However, an exemplary embodiment is not limited thereto. That is, the object 20 may lie down on the table 100 so that the chest of the object 20 faces an upper direction, that is, in the z-axis direction of the table 100. The positioning unit 102 may be formed to have a shape corresponding to the shape of the part that faces the upper surface of the table 100. For example, if the object 20 lies down on the table 100 so that the chest of the object 20 faces the upper surface of the table 100, a concave unit 104 corresponding to the shape of the chest of the object 20 is formed in an upper surface 106 of the positioning unit 102.

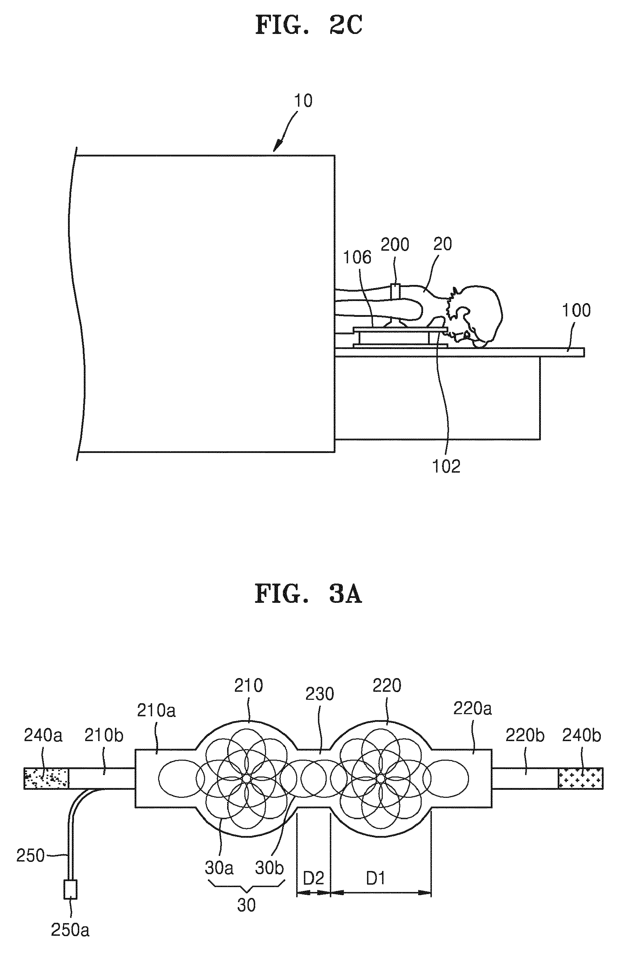

FIG. 2C is a schematic diagram showing the object 20 that is positioned on the table 100 of FIG. 2B and entering the bore 160.

Referring to FIGS. 1 and 2A through 2C, the object 20 may be positioned on the table 100 as lying down facing the surface of the table 100, and for diagnosis and inspection, the object 20 and the table 100 may enter the bore 160 of the cylindrical magnetic structure 10. The object 20 may be positioned on the positioning unit 102 of the table 100 while wearing the RF surface coil 200 on the chest. The chest of the object 20 may protrude from a surrounding region of an upper body of the object 20, and the protruding chest may be supported by the concave unit 104 of the positioning unit 102. The object 20 may enter into the bore 160 of the cylindrical magnetic structure 10 in while lying down on the table 100 and the positioning unit 102 of the table 100, and the capturing of a magnetic resonance image may be performed. To prevent or reduce a feeling of fatigue due to the process of capturing a magnetic resonance image, the upper surface 106 of the positioning unit 102 may be formed of a soft and cushioning material.

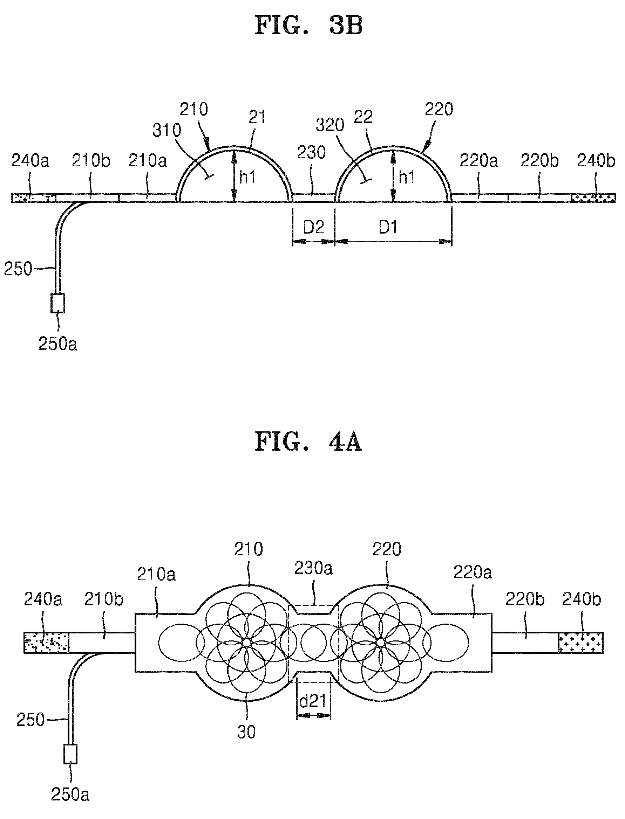

FIG. 3A is a bottom view of the RF surface coil 200, according to an exemplary embodiment. FIG. 3B is a cross-sectional view of the RF surface coil 200 of FIG. 3A.

Referring to FIGS. 3A and 3B, the RF surface coil 200 may include a first cup unit 210 and a second cup unit 220 that are formed to correspond to the chest of the object 20, and a center unit 230 between the first and second cup units 210 and 220. The first and second cup units 210 and 220 may have a shape to fit over the chest of the object 20, for example, a hemispherical shape. At least one RF coil element 30 may be formed in the first cup unit 210, the second cup unit 220, and the center unit 230 between the first and second cup units 210 and 220. The RF coil element 30 may be formed in an inner side of the first cup unit 210, the second cup unit 220, and the center unit 230. An RF coil element 30a formed in the first cup unit 210 and the second cup unit 220 and an RF coil element 30b formed in the center unit 230 may have an oval loop shape. However, an exemplary embodiment is not limited thereto, that is, the RF coil element 30 formed in the RF surface coil 200 may have a polygonal shape, a beam shape, or other shapes besides the oval shape.

The RF surface coil 200 may generate an RF magnetic field having a Larmor frequency as a center frequency on the chest of the object 20 through the RF coil element 30, may receive a magnetic resonance signal, that is, an RF signal emitted from the chest of the object 20, and may transmit the RF signal to a signal transceiver 12 of FIG. 1. For this purpose, each of the RF coil elements 30 may transmit the RF signal to the signal transceiver 12 through cables 250. Also, optionally, the RF surface coil 200 may further include a controller 250a that is connected to the RF coil element 30 through the cables 250, and allows or controls the RF coil element 30 to generate an RF magnetic field on a chest of the object 20 through the controller 250a and to receive an RF signal emitted from the chest of the object 20.

First extension units 210a and 210b and second extension units 220a and 220b are respectively formed on both sides of the first cup unit 210 and the second cup unit 220. A first assembling unit 240a and a second assembling unit 240b are respectively formed on edges of the first extension unit 210a and the second extension unit 220a. The first assembling unit 240a and the second assembling unit 240b may be coupled to each other so that the RF surface coil 200 is fixed on the upper body of the object 20. The first assembling unit 240a and the second assembling unit 240b may be assembled by various methods. For example, the method of assembling the first assembling unit 240a with the second assembling unit 240b may be a velcro assembling method, a button assembling method, or a hook assembling method, but is not limited thereto.

The first extension units 210a and 210b and the second extension units 220a and 220b may be respectively formed to have at least two regions. A first region 210a of the first extension units 210a and 210b and a first region 220a of the second extension units 220a and 220b are regions that are directly in contact with both lateral sides of the chest of the object 20, and as depicted in FIG. 3A, may be formed to have a relatively large width compared to a second region 210b of the first extension units 210a and 210b and a second region 220b of the second extension units 220a and 220b.

The RF surface coil 200 according to an exemplary embodiment may be formed of a soft material having flexibility so that a feeling of fatigue of the object 20 wearing the RF surface coil 200 is prevented or reduced in the process of capturing a magnetic resonance image for long hours. The first cup unit 210, the first extension units 210a and 210b, the second cup unit 220, the second extension units 220a and 220b, and the center unit 230 may be formed of a material, such as rubber or polymer, for example, and may be formed of a base material, such as flexible foam or urethane.

The RF surface coil 200 according to an exemplary embodiment may be a wearable type by being directly attached to a chest of the object 20. The chest of the object 20 may vary according to a gender of the object 20. If the object 20 is a female, the chest, that is, the size and circumference of a breast and a gap between the two breasts may differ. If the sizes of parts of the RF surface coil 200 are designed regardless of the body characteristics of the object 20, the quality reliability of the magnetic resonance image obtained by an RF signal may not be guaranteed.

The RF surface coil 200 according to an exemplary embodiment may be formed to control the sizes, that is, lengths and heights of the first cup unit 210, the first extension units 210a and 210b, the second cup unit 220, the second extension units 220a and 220b, and the center unit 230 to correspond to the body characteristics, that is, a circumference of an upper body and the sizes or volumes of the breasts of the object 20. The first cup unit 210, the first extension units 210a and 210b, the second cup unit 220, the second extension units 220a and 220b, and the center unit 230 of the RF surface coil 200 may be formed of rubber or polymer having flexibility and elasticity, and thus, a length may be controlled to match a circumference of the upper body of the object 20. Also, assembling locations of the first assembling unit 240a and the second assembling unit 240b may be controlled. For example, when the first assembling unit 240a and the second assembling unit 240b are formed of velcro, the assembling locations of the first assembling unit 240a and the second assembling unit 240b may be changed according to the circumference of the upper body of the object 20.

Circumferences, lengths (or diameter D1), and heights h1 of the first cup unit 210 and the second cup unit 220 may be controlled according to the sizes and circumferences of breasts of the object 20. Inner spaces 310 and 320 of the first cup unit 210 and the second cup unit 220, respectively, are controlled according to the size of the chest of the object 20, and thus, a magnetic resonance image may be captured while the chest of the object 20 is in contact with inner surfaces 21 and 22 of the first cup unit 210 and the second cup unit 220, respectively. Therefore, a magnetic resonance image of high quality may be obtained. A length D2 of the center unit 230 is controlled according to a gap between breasts of the object 20.

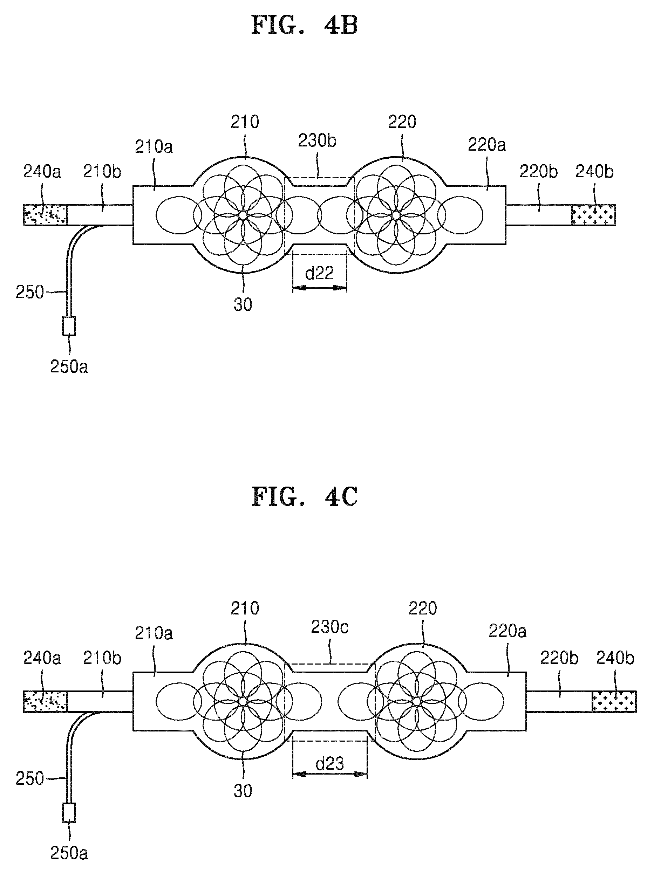

FIGS. 4A, 4B, and 4C are schematic diagrams of the RF surface coil 200 in which a length of the center unit 230 is controlled, according to an exemplary embodiment.

FIG. 4A shows a case in which a length d21 of a center unit 230a of the RF surface coil 200 according to an exemplary embodiment is relatively short. FIG. 4B is a case in which a length d22 of a center unit 230b of the RF surface coil 200 according to an exemplary embodiment is relatively long. FIG. 4C is a case in which a length d23 of a center unit 230c of the RF surface coil 200 according to an exemplary embodiment is relatively longest. The lengths D2, d21, d22, and d23 of the center units 230, 230a, 230b, and 230c, respectively, of the RF surface coil 200 may be controlled in various ways. Although, the lengths D2, d21, d22, and d23 of the center units 230, 230a, 230b, and 230c, respectively, are controlled, the RF coil element 30 for capturing a magnetic resonance image of the central region of the chest of the object 20 may be maintained as it is.

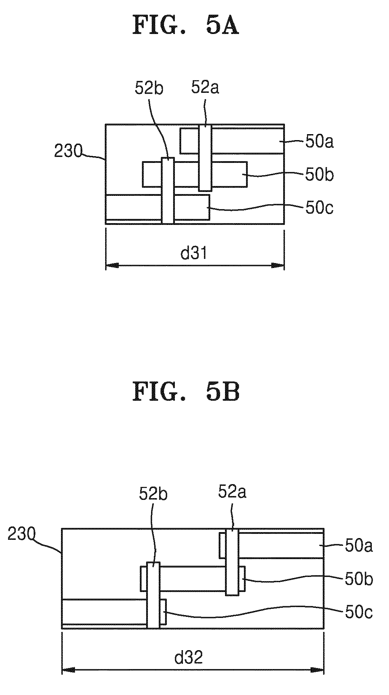

FIGS. 5A and 5B are schematic diagrams showing controlling a gap of the RF surface coil 200, according to an exemplary embodiment. FIG. 5A shows a case in which a length d31 of the center unit 230 of the RF surface coil 200 is relatively short, and FIG. 5B shows a case in which a length d32 of the center unit 230 of the RF surface coil 200 is relatively long.

Referring to FIGS. 5A and 5B, the center unit 230 of the RF surface coil 200 according to an exemplary embodiment may include at least two length control pieces 50a, 50b, and 50c. The length control pieces 50a, 50b, and 50c may be connected to each other by fixing bands 52a and 52b, and the fixing bands 52a and 52b may be coupled to the at least two length control pieces 50a, 50b, and 50c. The length of the center unit 230 of the RF surface coil 200 may be controlled according to coupling locations of the length control pieces 50a, 50b, and 50c with the fixing bands 52a and 52b. For example, as depicted in FIG. 5A, when the fixing band 52a is respectively connected to an edge of the first length control piece 50a and to an edge of the second length control piece 50b, the length of the center unit 230 may be formed to be long when compared to a case in which the fixing band 52a is connected to one of a center region of the first length control piece 50a and a center region of the second length control piece 50b. The length control pieces 50a, 50b, and 50c may be formed in plural and parallel to each other in the length directions d31 and d32. The length control pieces 50a, 50b, and 50c and the fixing bands 52a and 52b may be formed of a non-conductive material, and the material is not limited thereto. The length control pieces 50a, 50b, and 50c and the fixing bands 52a and 52b may be assembled in various ways, for example, a velcro assembling method, a button assembling method, or a hook assembling method, but the assembling method according to an exemplary embodiment is not limited thereto.

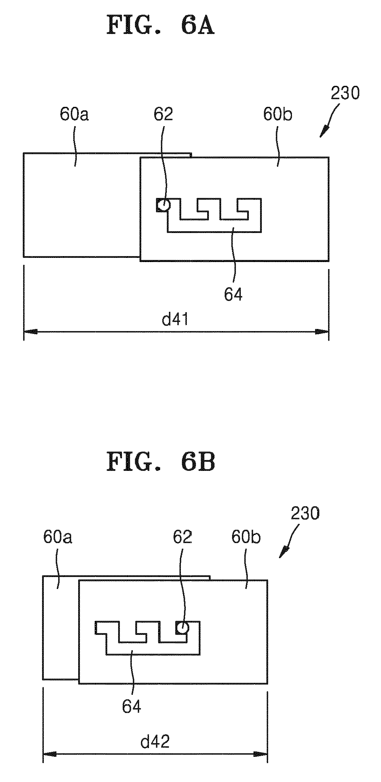

FIGS. 6A and 6B are schematic diagrams showing controlling a gap of the RF surface coil 200, according to another exemplary embodiment. FIG. 6A shows a case in which a length d41 of the center unit 230 of the RF surface coil 200 is relatively long, and FIG. 6B shows a case in which a length d42 of the center unit 230 of the RF surface coil 200 is relatively short.

Referring to FIGS. 6A and 6B, the center unit 230 of the RF surface coil 200 according to an exemplary embodiment may include a first region 60a and a second region 60b that are separated from each other. A position fixing protrusion 62 is formed on the first region 60a of the center unit 230, and a groove unit 64 in which the position fixing protrusion 62 is moveably inserted is formed on the second region 60b of the center unit 230. The groove unit 64 may be formed through the second region 60b of the center unit 230, and some of the groove unit 64 may be formed in the length direction (d41 and d42) of the center unit 230. The groove unit 64 in the second region 60b may include a first groove formed in the length direction of the center unit 230 and a second groove formed in a different direction from the length direction of the center unit 230. The position fixing protrusion 62 may be fixed in the second groove. The position fixing protrusion 62 is inserted in the groove unit 64 and moves in the length direction (d41 and d42) of the second region 60b, and thus, the length (d41 and d42) of the center unit 230 may be controlled.

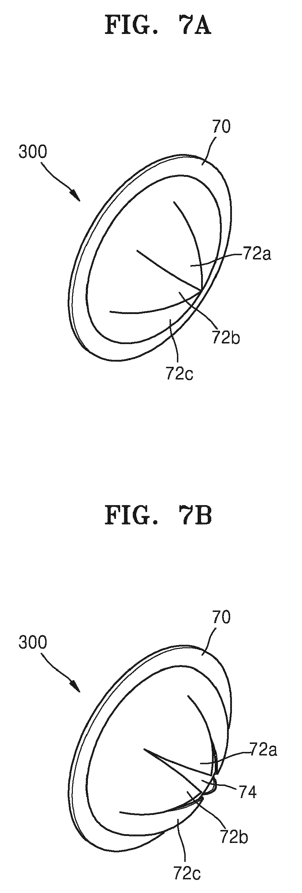

FIGS. 7A and 7B are perspective views of a cup 300 (the first cup unit 210 or the second cup unit 220) of the RF surface coil 200, according to an exemplary embodiment. FIG. 7A shows a case in which a chest of the object 20 is not in contact with the cup 300, and FIG. 7B shows a case in which the chest of the object 20 is in contact with the cup 300.

Referring to FIG. 7A, the cup 300 of the RF surface coil 200 includes a round unit 70 having a circular shape or an oval shape, and one or more cover units 72a, 72b, and 72c that are formed extending inwards from the round unit 70. The round unit 70 may be formed as a circular shape or an oval shape to surround a lower part of the chest of the object 20, and the cover units 72a, 72b, and 72c may be formed to surround an upper part of the chest of the object 20.

Referring to FIG. 7B, when the chest of the object 20 is in contact with the cup unit 300 of the RF surface coil 200, the cover units 72a, 72b, and 72c may be transformed according to the shape of the chest of the object 20 and may protrude outwards. The round unit 70 and the cover units 72a, 72b, and 72c may have a flexible characteristic. While contacting the chest of the object 20, the round unit 70 and the cover units 72a, 72b, and 72c may protrude according to the shape of the chest of the object 20, and regions between the cover units 72a, 72b, and 72c corresponding to the center region of the round unit 70 may be exposed. A cover unit 74 is formed on the center region of an inner side of the cover units 72a, 72b, and 72c. The cover units 72a, 72b, and 72c may be formed as one-body with the round unit 70, and may be formed as the same material as or a different material from the round unit 70. The round unit 70 may be formed of a flexible material or a non-flexible insulating material. In this case, an RF surface coil suitable for body shape (a body characteristic) of the object 20 may be selected by employing a plurality of cups 300 having various sizes (circumferences and widths).

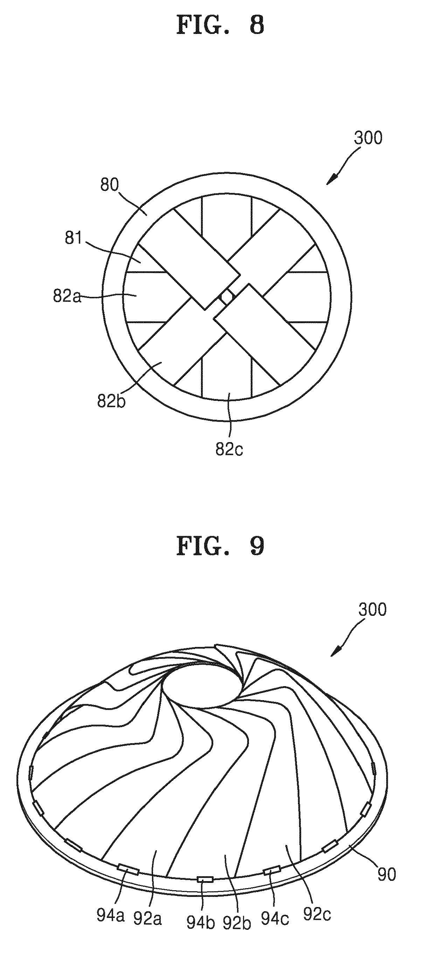

FIG. 8 is a schematic diagram of the cup 300 of the RF surface coil 200, according to another exemplary embodiment.

Referring to FIG. 8, the cup 300 of the RF surface coil 200 includes a round unit 80 having a circular shape or an oval shape and one or more cover units 82a, 82b, and 82c extending inwards from an inner side of the round unit 80. The round unit 80 may be formed as a circular shape or an oval shape to surround a lower part of the chest of the object 20, and may be formed of a flexible rubber or polymer. However, an exemplary embodiment is not limited thereto; that is, the round unit 80 may be formed in various sizes according to the body characteristics of the object 20. The cover units 82a, 82b, and 82c may be formed of a flexible material so that the shapes of the cover units 82a, 82b, and 82c are transformed when the cover units 82a, 82b, and 82c contact the chest of the object 20. In the case of FIG. 7A, the cover units 72a, 72b, and 72c are formed as one-body as an example in a region where the cover units 72a, 72b, and 72c and the round unit 70 contact each other. In FIG. 8, the cover units 82a, 82b, and 82c are individually combined with the round unit 80. The sizes and shapes of the cover units 82a, 82b, and 82c may be the same or different from each other, and a cover unit 81 having different shape is further included. The cover units 82a, 82b, and 82c may be formed to overlap each other.

FIG. 9 is a perspective view of the cup 300 of the RF surface coil 200, according to another exemplary embodiment.

Referring to FIG. 9, the cup 300 of the RF surface coil 200 includes a round unit 90 having a circular shape or an oval shape and one or more cover units 92a, 92b, and 92c extending inwards from an inner side of the round unit 90. The round unit 90 may be formed as a circular shape or an oval shape to surround a lower part of the chest of the object 20. The round unit 90 may be formed as various sizes according to a body characteristic of the object 20. The cover units 92a, 92b, and 92c may be formed of a flexible material. However, an exemplary embodiment is not limited thereto, that is, the cover units 92a, 92b, and 92c may be formed of a non-flexible material. For example, the cover units 92a, 92b, and 92c may be formed of a non-flexible material, such as plastic, polymer, or rubber, and may be formed in a type that the cover units 92a, 92b, and 92c overlap with each other. When the chest of the object 20 contacts the cover units 92a, 92b, and 92c, the cover units 92a, 92b, and 92c may protrude outwards by a force of contacting the chest of the object 20 in the overlapping state. The round unit 90 and the cover units 92a, 92b, and 92c of the RF surface coil 200 may be connected to each other in various ways. For example, the cover units 92a, 92b, and 92c may be connected to the round unit 90 by using respective hinges 94a, 94b, and 94c.

As described above, it is depicted that the cup 300 of the RF surface coil 200 according to an exemplary embodiment includes a round unit and cover units extending from the round unit. However, an exemplary embodiment is not limited thereto, that is, the cup may include a cover unit that is transformed according to the shape of the chest of the object 20 without including an additional round unit. The cover unit may be formed as a single unit or a plurality of units, and when the cover unit is formed as plural units, the individual cover units may be formed as one-body.

As described above, the MRI system according to an exemplary embodiment may include the wearable type RF surface coil 200 so that the RF surface coil 200 is directly contacting the chest of the object 20. The RF surface coil 200 may be transformed to various shapes according to a body type of the object 20, that is, the size of a breast, e.g., magnitude, volume, and gaps between the breasts. The RF surface coil 200 is transformed according to the body type of the object 20 by directly contacting the chest of the object 20, and thus, the degree of fatigue during capturing a magnetic resonance image may be minimized or reduced. Also, a high quality magnetic resonance image may be obtained.

Again referring to FIG. 1, a display 150 is mounted on an external of the housing 110 of the MRI system according to an exemplary embodiment. Also, an additional display to be viewed by the object 20 may further be mounted on an inner side of the housing 110. Information may be transmitted to the user or the object 20 through the displays located on inside and/outside of the housing 110.

The MRI system according to an exemplary embodiment includes the signal transceiver 12, a system controller 14, an operator 16, and a monitor 18. The transceiver 12 may control a gradient magnetic field formed in the bore 160 of the housing 110 where the object 20 is located. Also, the transceiver 12 may control the transmission and receiving of an RF signal and a magnetic resonance signal with respect to the RF body coil 140 and the RF surface coil 200. The system controller 14 may control sequence of signals formed in the housing 110. The monitor 18 may monitor and control the housing 110 and various devices mounted in the housing 110. The operator 16 may control an overall operation of the MRI system through the system controller 14. The object 20, while wearing the RF surface coil 200, may be located on the positioning unit 102 of the table 100, and may be moved in the direction of the bore 160, that is, in the y-direction by the movement of the table 100. Thus, an image capturing operation of MRI for diagnosis and inspection of the object 20 may be performed in a paused state or a moving state.

According to an exemplary embodiment, an RF surface coil, a shape and a size thereof are controlled according to the shapes and sizes of chests of objects. Also, an MRI system is provided that includes the RF surface coil, a shape and a size thereof that are controlled according to the shapes and sizes of chests of objects.

The degree of contact between a chest of an object and an RF surface coil is increased, and thus, a high receiving sensitivity is obtained. As a result, a high quality magnetic resonance image may be obtained. Also, because the RF surface coil is controlled in accordance with the shape and size of the chest of the object, the feeling of fatigue of the object may be lowered.

The foregoing exemplary embodiments and advantages are examples and are not to be construed as limiting. The present teaching may be readily applied to other types of apparatuses. Also, the description of the exemplary embodiments is intended to be illustrative, and not to limit the scope of the claims, and many alternatives, modifications, and variations will be apparent to those skilled in the art.

## Claims

1. A radio frequency (RF) surface coil for a magnetic resonance imaging apparatus, the RF surface coil comprising:
a first cup unit and a second cup unit corresponding to a chest of an object, the first cup unit and the second cup unit being transformable to a shape of the chest; and
an RF coil element disposed on the first cup unit and the second cup unit.

2. The RF surface coil of claim 1, further comprising a center unit disposed between the first cup unit and the second cup unit,

3. The RF surface coil of claim 2, wherein the RF coil element is further disposed on the center unit,
wherein the RF coil element is preferably disposed on an inner surface of the first cup unit, the second cup unit, and the center unit.

4. The RF surface coil of claim 3, further comprising a cable connected to the RF coil element.

5. The RF surface coil of any of claims 1-4, further comprising a controller connected to the cable, the controller being configured to control the RF coil element to generate an RF magnetic field on the chest of the object, and receive an RF signal that is emitted from the chest,
wherein preferably a length of the center unit is adjustable.
wherein the center unit preferably comprises length control pieces and fixing bands, the length control pieces being fixable by the fixing bands.

6. The RF surface coil of any of claims 2-5, wherein the center unit comprises a first region and a second region, the first region being separated from the second region.

7. The RF surface coil of claim 6, further comprising:
a position fixing protrusion disposed in the first region; and
a groove disposed in the second region, the position fixing protrusion being moveably inserted in the groove,
wherein the groove comprises a first groove in a direction of a length of the center unit, and a second groove in a direction different from the direction of the length of the center unit.

8. The RF surface coil of any of claims 1-7, further comprising:
a first extension unit disposed on an edge of the first cup unit;
a second extension unit disposed on an edge of the second cup unit;
a first assembling unit disposed on an edge of the first extension unit; and
a second assembling unit disposed on an edge of the second extension unit.

9. The RF surface coil of claim 8, wherein at least one among the first extension unit and the second extension unit comprises a first region and a second region, the first region having a width different than a width of the second region.

10. The RF surface coil of claim 9, wherein the first assembling unit is coupled to the second assembling unit,
wherein the first assembling unit is preferably coupled to the second assembling unit, using one among velcro, a button, and a hook.

11. The RF surface coil of any of claims 1-10, wherein at least one among the first cup unit and the second cup unit comprises a cover unit transformable according to a shape of the chest of the object.

12. The RF surface coil of claim 16, wherein the at least one among the first cup unit and the second cup unit comprises a round unit from which the cover unit extends.
wherein at least one among the cover unit and the round unit preferably comprises a flexible material, and/or
wherein the cover unit and the round unit are one-body, and/or
wherein the cover unit comprises cover units, and
the cover units and the round unit are one-body, and/or
wherein the at least one among the first cup unit and the second cup unit comprises another cover unit disposed on a center region of an inner surface of the cover units, and/or
wherein the cover unit comprises cover units that are individually connected to the round unit, and/or
wherein the cover unit comprises a non-flexible material, and/or
wherein the at least one among the first cup unit and the second cup unit comprises a hinge connecting the cover unit to the round unit.

13. A magnetic resonance imaging (MRI) apparatus comprising:
a cylindrical magnetic structure;
a table on which an object is positioned, the table being configured to be in the cylindrical magnetic structure; and
a radio frequency (RF) surface coil comprising:
a first cup unit and a second cup unit corresponding to a chest of the object, the first cup unit and the second cup unit being transformable to a shape of the chest; and
an RF coil element disposed on the first cup unit and the second cup unit.

14. The MRI apparatus of claim 13, wherein the RF surface coil further comprises a center unit disposed between the first cup unit and the second cup unit.

15. The MRI apparatus of claim 14, holding the RF surface coil according to any of claims 1-12.
